# EUROPEAN PATENT APPLICATION

(11) **EP 4 116 413 A1**
(43) Date of publication of application: **11.01.2023**
(21) Application number: 21764927.6
(22) Date of filing: 26.02.2021
(51) Int. Cl.: C12N 5/0775, C12N 1/20, C12N 5/00, C12R 1/225

(54) **COMPOSITION FOR REGULATING STEM CELL FUNCTION COMPRISING LACTOBACILLUS SP. BACTERIA-DERIVED EXTRACELLULAR VESICLES AS ACTIVE INGREDIENT**

(30) Priority: 04.03.2020 KR 20200027386
(71) Applicant: MD Healthcare Inc., Seoul 03923 (KR); Chungbuk National University Industry-Academic Cooperation Foundation, Cheongju-si, Chungcheongbuk-do 28644 (KR)
(72) Inventor: KIM, Yoon-Keun, Paju-Si, Gyeonggi-do 10908 (KR); PARK, Yoon Shin, Cheongju-si, Chungcheongbuk-do 28415 (KR); CHOI, Da Hyeon, Cheongju-si, Chungcheongbuk-do 28645 (KR)
(74) Representative: Dehns
(86) International application number: PCT/KR2021/002511
(87) International publication number: WO 2021/177680

(57) **Abstract**

The present invention relates to a composition for regulating stem cell function comprising the genus *Lactobacillus* bacteria-derived extracellular vesicles as an active ingredient, and a method for regulating a stem cell function using the same. The genus *Lactobacillus* bacterial-derived extracellular vesicles, according the present invention, have the effect of greatly improving the proliferation of stem cells, and the extracellular vesicles are naturally secreted from a symbiotic the genus *Lactobacillus* strain in human tonsillar tissue and are human-friendly and natural substances, and thus can be used in various research fields by promoting the proliferation of aged stem cells with minimal toxicity and side effects.

## Description

### [Technical Field]

The present invention relates to a composition for regulating stem cell function, which comprises the genus *Lactobacillus* bacteria-derived extracellular vesicles (EVs) as an active ingredient, and a method of regulating stem cell function using the same.

This application claims priority to and the benefit of Korean Patent Application No. 10-2020-0027386, filed on March 4, 2020, and all the contents disclosed in the specification and drawings of the applications are incorporated in this application.

### [Background Art]

Recently, from archaea, bacteria, viruses to eukaryotes such as yeast and mold, microorganisms maintain a symbiotic relationship with humans by acquiring various ecological niches inside and outside the human body. The number of microorganisms in the human body is more than 10 times the total number of human cells, and the number of genes possessed by microorganisms is also several hundred times the human genome. It is known that these microorganisms perform major functions through interactions with the human body, such as the absorption and metabolism of nutrients in the human body, the maturation and development of the immune system and the nervous system, and influencing the occurrence and prevention of various diseases. As such, a human is a super-organism which has a mixture of cellular and genetic characteristics of numerous symbiotic microorganisms, in addition to human cells and genome, and the collection of all microorganisms that are naturally present in the human body is called a microbiome.

The human microbiome is dynamically changed due to various factors such as an individual, family, race, gender, age, diet, region, and lifestyle. However, it was found that, unlike the diversity of microbial species constituting each individual's microbial community, the core functional genes constituting the microbiome are common for the most part despite the difference in individual characteristics and environmental conditions.

Therefore, human-derived adult stem cells may also undergo changes in stemness, which is the unique characteristic of stem cells, according to the action of the microbiome. Various stem cell studies using adult stem cells are being actively conducted, and for the development of cell therapeutics using adult stem cells, it is necessary to identify a microbiome that affects stem cells. In fact, multi-modal studies on the extracellular vesicles (EVs), which are substances secreted from various microbiomes for general cells, are ongoing, but there is no such thing as a clear indicator substance.

### [Disclosure]

### [Technical Problem]

The present invention is provided to solve the above-described problems in the related art, and the present invention is directed to providing a composition for regulating stem cell function, comprising the genus *Lactobacillus* bacteria-derived extracellular vesicles (EVs) as an active ingredient.

The present invention is also directed to providing a method of regulating stem cell function, comprising treating a stem cell culture medium with the genus *Lactobacillus* bacteria-derived extracellular vesicles (EVs).

However, a technical problem to be achieved by the present invention is not limited to the aforementioned problems, and the other problems that are not mentioned may be clearly understood by a person skilled in the art from the following description.

### [Technical Solution]

To achieve the purpose of the present invention, the present invention provides a composition for regulating stem cell function, comprising the genus *Lactobacillus* bacteria-derived extracellular vesicles (EVs) as an active ingredient.

In one embodiment of the present invention, the genus *Lactobacillus* bacteria may comprise, for example, *Lactobacillus paracasei, Lactobacillus plantarum, Lactobacillus rhamnosus,* and the like, but there is no limitation on the species.

In another embodiment of the present invention, the genus *Lactobacillus* bacteria may be derived from the human body, but the present invention is not limited thereto.

In one embodiment of the present invention, the stem cell may be a mesenchymal stem cell, but the present invention is not limited thereto.

In another embodiment of the present invention, the stem cell may be a tonsil-derived stem cell, but the present invention is not limited thereto.

In still another embodiment of the present invention, the composition may promote the proliferation of a stem cell.

In yet another embodiment of the present invention, the concentration of extracellular vesicles (EVs) may be 10 to 1000 µg/ml, but the present invention is not limited thereto.

In yet another embodiment of the present invention, the EVs may have an average diameter of 10 to 200 nm, but the present invention is not limited thereto.

In yet another embodiment of the present invention, the EVs may have a zeta potential of -60 to 30 mV, but the present invention is not limited thereto.

In yet another embodiment of the present invention, the EVs may be naturally or artificially secreted from the genus *Lactobacillus* bacteria.

In addition, the present invention provides a method of regulating stem cell function, comprising treating a stem cell culture medium with the genus *Lactobacillus* bacteria-derived extracellular vesicles.

In one embodiment of the present invention, the culture medium may be selected from the group consisting of DMEM medium, DMEM/F12 medium, M199/F12 mixture, MEM-α medium, MCDB 131 medium, IMEM medium, PCM medium, and MSC expansion medium, but the present invention is not limited thereto.

In another embodiment of the present invention, the method may be to regulate the proliferation rate of stem cells.

### [Advantageous Effects]

The present invention relates to a composition for regulating stem cell function, comprising the genus *Lactobacillus* bacteria-derived extracellular vesicles (EVs) as an active ingredient, and a method of regulating stem cell function, comprising treating a stem cell culture medium with the genus *Lactobacillus* bacteria-derived extracellular vesicles (EVs). By using the composition or method according to the present invention, there is an effect of greatly improving the proliferative potential of a stem cell. In addition, the EVs are naturally secreted from a symbiotic the genus *Lactobacillus* strain in human tonsillar tissue and may be a human-friendly and natural substance compared to the promotion of stem cell proliferation by the treatment with a chemical drug. Therefore, aged stem cells, which have lost their value as stem cells, can have enhanced proliferation with minimal toxicity and side effects and are expected to be used in various research fields.

### [Description of Drawings]

FIGS. 1A and 1B show the morphology and size of the genus *Lactobacillus* bacteria-derived extracellular vesicles, in which FIG. 1A is an image of the genus *Lactobacillus* bacteria-derived extracellular vesicles obtained using a transmission electron microscope, and FIG. 1B shows a graph showing the average particle size of the vesicles.
FIG. 2 is a graph obtained by measuring the zeta potential of the genus *Lactobacillus* bacteria-derived extracellular vesicles in DMEM which is a stem cell culture medium.
FIG. 3 is an image obtained using an optical microscope to compare the degree of proliferation for the same time period after stem cells are treated with the genus *Lactobacillus* bacteria-derived extracellular vesicles at 100, 200, 300, 400, or 500 µg/ml.
FIG. 4 is a graph obtained by measuring the degree of proliferation every 3 hours for 42 hours after stem cells are treated with the genus *Lactobacillus* bacteria-derived extracellular vesicles at 100, 200, 300, 400, or 500 µg/ml.

### [Modes of the Invention]

As a result of making an effort to find microbiome extracellular vesicles affecting stem cells, the inventors confirmed that the genus *Lactobacillus* bacteria-derived extracellular vesicles affect the proliferation potential and stemness of human tonsillar tissue-derived stem cells, and the present invention was completed.

Thus, the present invention may provide a composition for regulating stem cell function, comprising the genus *Lactobacillus* bacteria-derived extracellular vesicles (EVs) as an active ingredient.

As used herein, the term "extracellular vesicle" refers to a structure formed of a nano-sized membrane secreted from various bacteria. Vesicles derived from gram-positive bacteria such as *Lactobacillus* include peptidoglycan and lipoteichoic acid, which are components of the bacterial cell wall, and various low-molecular compounds in the vesicle, as well as proteins and nucleic acids.

There is no limitation on the genus *Lactobacillus* bacterial species. For example, *L. paracasei, L. plantarum, L. rhamnosus, L. acidophilus, L. amylovorus, L. casei, L. brevis, L. crispatus, L. delbrueckii* subsp. *lactis, L. bulgaricus, L. fermentum, L. helveticus, L. gallinarum, L. gasseri, L. johnsonii, L. reuteri, L. salivarius, L. alimentarius, L. curvatus, L. casei* subsp. *casei,* and *L sake* may be included in the scope of the present invention.

The genus *Lactobacillus* bacteria of the present invention may be derived from the human body, and preferably may be, a *Lactobacillus* strain derived from the same tissue as tissue from which stem cells are derived. For example, to control human tonsillar tissue-derived mesenchymal stem cell function, a symbiotic *Lactobacillus* strain in the human tonsil may be isolated, and extracellular vesicles secreted therefrom may be isolated, or extracellular vesicles artificially isolated by culturing the isolated *Lactobacillus* strain may be used. However, the origin of *Lactobacillus* bacteria is not limited to being derived from specific tissue, but the present invention is not limited thereto.

The genus *Lactobacillus* bacteria-derived EVs of the present invention may be naturally or artificially secreted, or may be isolated from the culture of the genus *Lactobacillus* bacteria or food fermented with the genus *Lactobacillus* bacteria. A method of isolating EVs from the cell culture or the bacteria-added fermented food may be isolating EVs from the culture or fermented food which includes EVs using centrifugation, ultra-high-speed centrifugation, filtration through a filter, gel filtration chromatography, free-flow electrophoresis, capillary electrophoresis, and a combination thereof, but the present invention is not limited thereto. In addition, processes such as removing impurities and concentration of the acquired EVs may be further included.

The EVs isolated by the above method may have an average diameter of 10 to 200 nm, and preferably 50 to 150 nm, but the present invention is not limited thereto. In addition, the EV particles may have a zeta potential of -60 to 30 mV, and since most particles have a zeta potential of -30 to -20 mV, they may be stably dispersed in a stem cell medium such as DMEM.

The term "function regulation" used herein refers to an action affecting the functions of all stem cells, including the proliferative potential of stem cells, by treating the stem cells with the composition according to the present invention. Particularly, the composition according to the present invention may have an effect of dramatically improving the proliferative potential of stem cells, and it was shown that the proliferative potential is improved even when stem cells lose stemness due to aging (see Example 3). Therefore, the "function" used herein preferably refers to the proliferative potential of stem cells, and the "regulation" preferably refers to control of the proliferation rate of stem cells, but the present invention is not limited thereto.

The term "proliferation" used herein refers to an increase in cell count. According to an exemplary embodiment of the present invention, it was confirmed that, when the same amount of stem cells are treated with the genus *Lactobacillus* bacteria-derived EVs, growth and proliferation rates are promoted.

The term "stem cell" used herein refers to a cell with pluripotency which allows the cell to differentiate into endoderm, mesoderm and ectoderm-derived animal cells and limited differentiation potential (multipotency) which allows the cell to differentiate into closely related cells. The stem cells that can be used for the purpose of the present invention may be included without limitation.

The type of stem cell to which a composition for regulating stem cell function according to the present invention can be applied is not limited, but in one embodiment of the present invention, the stem cells may be mesenchymal stem cells derived from the tonsils, bone marrow, umbilical cord blood or adipose tissue. The mesenchymal stem cells may be isolated from mammals, such as a human, a mouse, a rat, a guinea pig, a rabbit, a monkey, a pig, a horse, a cow, a sheep, an antelope, a dog or a cat, but the present invention is not limited thereto. The mesenchymal stem cells are preferably isolated from a human. A method of acquiring such mesenchymal stem cells is well known in the art.

As another aspect of the present invention, the present invention may provide a method of regulating stem cell function, comprising treating a stem cell culture medium with the genus *Lactobacillus* bacteria-derived extracellular vesicles.

The stem cell culture medium is a known basic medium commonly used for cell culture, which may be, for example, Dulbecco's modified Eagle's medium (DMEM), Dulbecco's modified Eagle's medium/Ham's F-12 (DMEM/F12), an M199/F12 mixture, alpha minimal essential medium (MEM-α), MCDB 131 medium, IMEM medium, PCM medium, or MSC expansion medium, but the present invention is not limited thereto.

These basic media contain a salt, a vitamin, a buffer, an energy source, an amino acid, and other substances, and when culturing stem cells, contain approximately 10 to 30% of animal-derived serum to provide universal nutrients for the growth of cells to be cultured.

The serum used in the culture of stem cells may be fetal, calf, horse or human serum, and preferably, fetal bovine serum (FBS). In addition, a compound having a component similar to that of animal-derived serum, for example, a bovine pituitary extract (BPE), may be used.

In addition, when culturing stem cells, it is preferable to add an antibiotic, an antifungal agent, and a reagent for preventing the growth of mycoplasma causing contamination. As an antibiotic, one that is used in common cell culture, such as penicillin, streptomycin or fungizone may be used. It is preferable to use amphotericin B as an antifungal agent and tylosin as a mycoplasma inhibitor, and gentamicin, ciprofloxacin, or azithromycin may be used to prevent mycoplasma contamination.

An oxidative nutrient such as L-glutamine and an energy metabolite such as sodium pyruvate may be further added as necessary.

The proliferation rat of stem cells may be regulated by treating the medium for stem cell culture with the genus *Lactobacillus* bacteria-derived EVs according to the present invention at a suitable concentration.

Terms and words used in the specification and claims should not be construed as being limited to general or dictionary terms meanings, and should be interpreted with the meaning and concept in accordance with the technical idea of the present invention based on the principle that the inventors have appropriately defined the concepts of terms in order to explain the invention in the best way.

Hereinafter, preferred Examples for helping the understanding of the present invention will be suggested. However, the following Examples are provided only to more easily understand the present invention, and the contents of the present invention are not limited by the following Examples.

### [Examples]

### Example 1: Screening of Lactobacillus strain

A *Lactobacillus paracasei* strain expected to have efficacy on tonsillar tissue-derived stem cells was screened by examining a microbiome in tonsillar tissue in the human oral cavity.

### Example 2: Characterization of isolated EVs

To analyze the characteristics of the genus *Lactobacillus* bacteria-derived EVs isolated in Example 1, the size and morphology of EV particles were observed using transmission electron microscopy, the zeta potential of EVs was measured in a medium for stem cell culture, Eagle's minimal essential medium (DMEM), to analyze repulsion or attraction between particles.

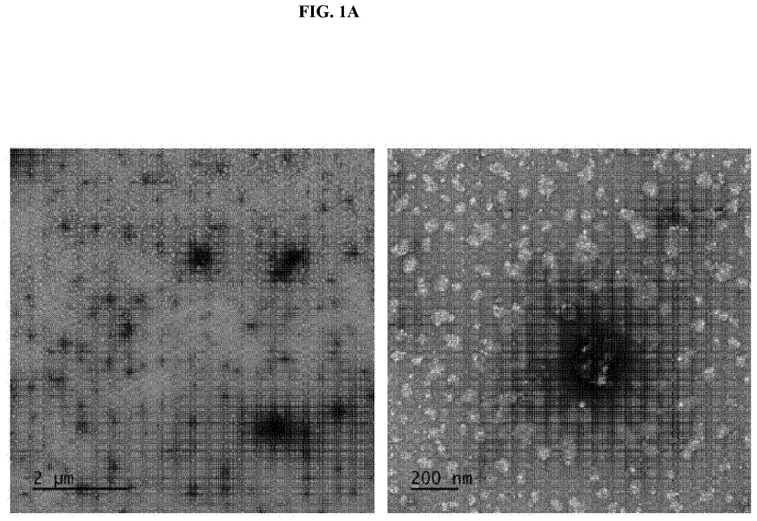

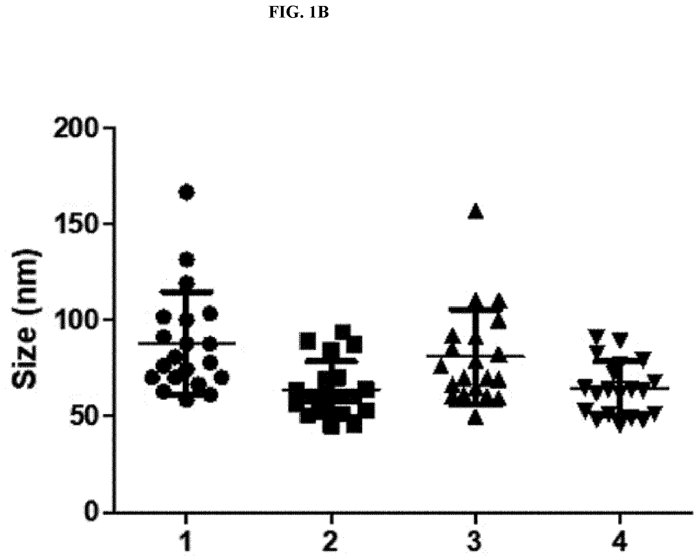

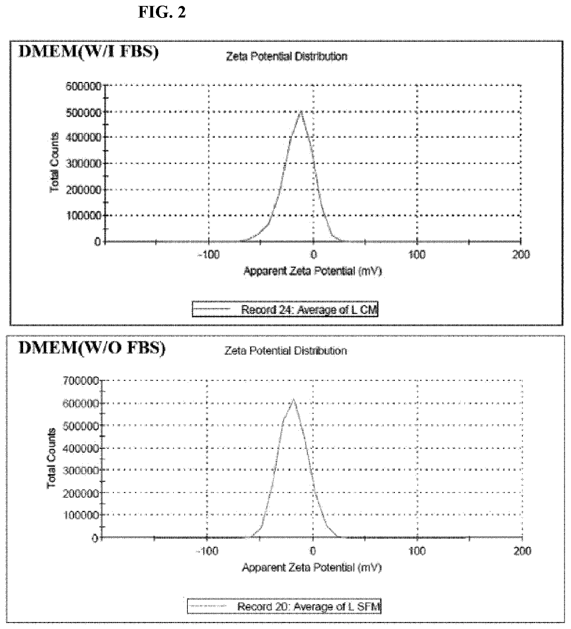

As a result, as shown in FIGS. 1A and 1B, it was confirmed that the genus *Lactobacillus* bacteria-derived EVs have a size of 60 to 150 nm on average. In addition, as shown in FIG. 2, it was confirmed that the zeta potential of almost all vesicle particles ranges from -60 to 30 mV This result shows that the isolated EVs do not aggregate in the medium and are appropriately dispersed and stably present.

### Example 3: Confirmation of effect of promoting proliferation potential of stem cells according to treatment of the genus Lactobacillus bacteria-derived extracellular vesicles by concentration

After stem cells were treated with the genus *Lactobacillus* bacteria-derived EVs isolated in Example 1 at a concentration of 100, 200, 300, 400 and 500 µg/ml, the degrees of stem cell proliferation were confirmed for the same time period.

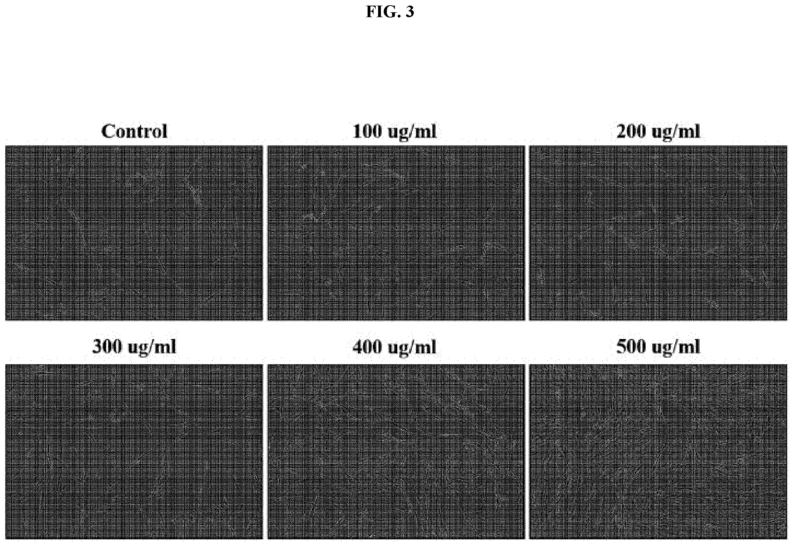

As a result, as shown in FIG. 3, as a result of comparing the amounts of proliferation of tonsil-derived stem cells for the same time period, it was able to be confirmed that proliferation most actively occurs in a group treated with 500 µg/ml of the genus *Lactobacillus* bacteria-derived EVs.

In addition, after stem cells with significantly reduced proliferative potential due to aging were treated with 100, 200, 300, 400 and 500 µg/ml of the genus *Lactobacillus* bacteria-derived EVs, cell proliferation saturation was measured for 42 hours at 3-hour intervals.

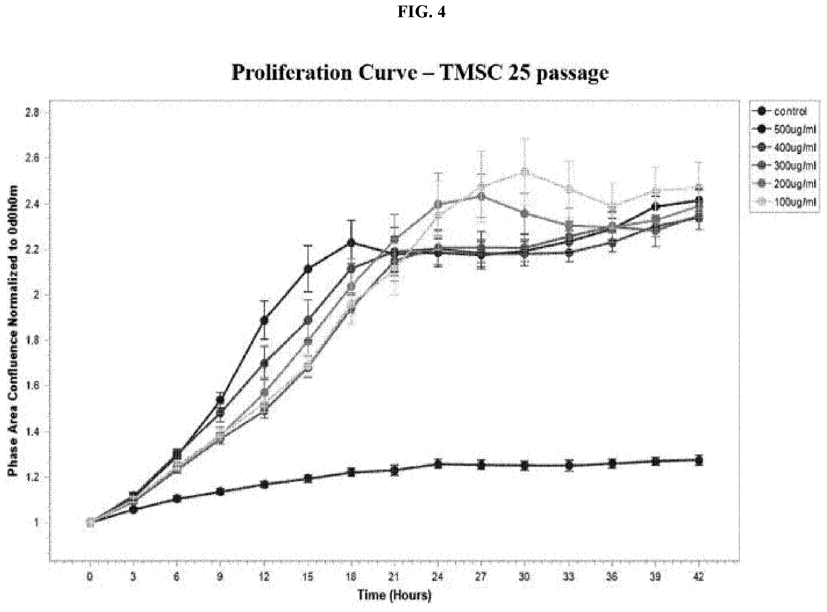

As a result, as shown in FIG. 4, compared with a control, it was shown that the proliferation of stem cells is significantly promoted at all concentrations in the group treated with the genus *Lactobacillus* bacteria-derived EVs. Particularly, when the EVs were treated at the highest concentration of 500 µg/ml, the fastest proliferation pattern was shown for the first 18 hours, and interestingly, even when the EVs were treated at the lowest concentration of 100 µg/ml, at 21 hours after treatment, compared with the groups treated with 200 µg/ml or more of the EVs, equivalent proliferative potentials were shown.

From the above results, to rapidly proliferate stem cells from the beginning, the stem cells may be treated with 400 to 500 µg/ml of the genus *Lactobacillus* bacteria-derived EVs, and even when the stem cells were treated with 100 µg/ml of the genus *Lactobacillus* bacteria-derived EVs, after approximately a day, it was able to be seen that the proliferation of stem cells was promoted to the same level as the case of treatment with a concentration of 500 µg/ml.

Particularly, even when stem cells losing stemness and showing below average growth rates due to aging were treated with the genus *Lactobacillus* bacteria-derived EVs, it was confirmed that proliferation was significantly improved.

The above-described description of the present invention is provided for illustrative purposes, and those of ordinary skill in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [Industrial Applicability]

The present invention relates to a composition for regulating stem cell function, comprising the genus *Lactobacillus* bacteria-derived EVs as an active ingredient, and the proliferative potential of stem cells may be greatly improved using the composition according to the present invention. In addition, the EVs are naturally secreted from a symbiotic the genus *Lactobacillus* strain in human tonsillar tissue, and since the EVs are human-friendly natural substances, compared to the promotion of stem cell proliferation by treatment with a chemical drug, they are considered to have high industrial usefulness as the proliferation of aged stem cells that have lost their value as stem cells is promoted with minimal toxicity and side effects and thus the EVs can be used in various research fields.

## Claims

1. A composition for regulating stem cell function, comprising the genus *Lactobacillus* bacteria-derived extracellular vesicles (EVs) as an active ingredient.

2. The composition of claim 1, wherein the genus *Lactobacillus* bacteria is derived from the human body.

3. The composition of claim 1, wherein the stem cell is a mesenchymal stem cell.

4. The composition of claim 1, wherein the stem cell is a tonsil-derived stem cell.

5. The composition of claim 1, wherein the composition promotes the proliferation of a stem cell.

6. The composition of claim 1, wherein the concentration of extracellular vesicles (EVs) is 10 to 1000 µg/ml.

7. The composition of claim 1, wherein the EVs have an average diameter of 10 to 200 nm.

8. The composition of claim 1, wherein the EVs are naturally or artificially secreted from the genus *Lactobacillus* bacteria.

9. A method of regulating stem cell function, comprising treating a stem cell culture medium with the genus *Lactobacillus* bacteria-derived extracellular vesicles.

10. The method of claim 9, wherein the culture medium is selected from the group consisting of DMEM medium, DMEM/F12 medium, M199/F12 mixture, MEM-α medium, MCDB 131 medium, IMEM medium, PCM medium, and MSC expansion medium.

11. The method of claim 9, wherein the method is to regulate the proliferation rate of stem cells.

12. The composition of claim 1, wherein the genus *Lactobacillus* bacteria is *Lactobacillus paracasei.*

13. The method of claim 9, wherein the genus *Lactobacillus* bacteria is *Lactobacillus paracasei.*
